# EUROPÄISCHE PATENTSCHRIFT

(11) **EP 1 062 963 B1**
(45) Veröffentlichungstag und Bekanntmachung des Hinweises auf die Patenterteilung: **04.08.2004**
(21) Anmeldenummer: 00110679.8
(22) Anmeldetag: 19.05.2000
(51) Int. Cl.: A61M 16/04

(54) **Vorrichtung zum Fixieren eines Endotrachialtubus**
Device for securing an endotracheal tube
Dispositif pour fixer un tube endotrachéal

(30) Priorität: 22.06.1999 DE 19928487
(43) Veröffentlichungstag der Anmeldung: 27.12.2000
(73) Patentinhaber: Molnar, Zoltan, 72510 Stetten am kalten Markt (DE)
(72) Erfinder: Molnar, Zoltan, 72510 Stetten am kalten Markt (DE)
(74) Vertreter: Möbus, Daniela, Dr.-Ing.

(56) Entgegenhaltungen:
- DE-A- 19 730 693
- DE-U- 9 015 001
- US-A- 4 498 903
- US-A- 5 894 840

## Beschreibung

Die Erfindung betrifft eine Vorrichtung zum Fixieren eines Endotrachialtubus am Kopf eines Patienten mit einer über den Mund des Patienten legbaren Platte, die eine Durchgangsöffnung für den Endotrachialtubus und eine weitere Durchgangsöffnung zur Fixierung eines Guedeltubus aufweist, wobei der Endotrachialtubus mittels eines Befestigungselements in der Durchgangsöffnung fixierbar ist.

Guedeltuben und Endotrachialtuben werden bisher meist mittels Pflastern oder Bändern am Kopf des Patienten festgelegt, was aufwändig und wenig sicher ist. Deshalb kann es passieren, dass sich die Tubuslage während des Beatmungsvorgangs verändert, sodass Beatmungskomplikationen mit Gefährdung des Patienten auftreten können.

Bei einigen der bekannten Vorrichtungen wird der Endotrachialtubus mittels eines Pflasters an einem mit der über den Mund des Patienten legbaren Platte verbundenen Steg befestigt. Wenn nun der Endotrachialtubus zu weit in die Luftröhre geschoben wurde, sodass nur eine Lungenhälfte beatmet wird, muss man das Pflaster mühsam entfernen, den Endotrachialtubus entsprechend weit aus der Luftröhre herausziehen und dann den Endotrachialtubus mit einem Pflaster erneut am Steg fixieren. Dies ist eine sehr aufwändige Prozedur, die eine Notfallsituation verursachen kann. Um diesem Problem abzuhelfen, wurde eine über den Mund des Patienten legbare Platte entwickelt, bei der der Endotrachialtubus mittels eines Schraubmechanismus an der Platte befestigt ist und somit mittels eines einfachen Handgriffes durch öffnen und Schließen des Schraubmechanismus schnell in seiner Lage korrigiert werden kann. Nachteilig bei dieser Lösung ist jedoch, dass die Schraube gegen ein unbeabsichtigtes Öffnen, beispielsweise durch Kopfbewegungen des Patienten, nicht gesichert ist. Somit kann sich der Endotrachialtubus unerwünschterweise in seiner Position verändern, wodurch Komplikationen auftreten können.

Aus der DE 90 15 001 U1 ist eine Halterung für Endotrachialtuben bekannt, die eine ovalförmige flexible Kunststoffplatte aufweist, in der eine Öffnung für einen Endotrachialtubus und eine weitere Öffnung vorgesehen ist, wobei die eine Öffnung mit einem sich zu einer Plattenkante erstreckenden Schlitz in Verbindung steht. Durch den Schlitz kann ein Tubus in die Öffnung eingeführt werden. Durch Einhaken eines Verschlussbügels wird der Tubus fest umschlossen.

Aus der US 4,498,903 ist ein Befestigungsmittel für einen Tubus bekannt geworden, das zwei Hälften aufweist, die auseinander klappbar sind. Zwischen den Hälften ist eine Durchgangsöffnung ausgebildet, in die ein Tubus eingelegt werden kann. Die beiden Hälften sind miteinander verrastbar.

Aus der US 5,894,840 ist eine Haltevorrichtung für einen Endotrachialtubus bekannt geworden, die ein Band aufweist, welches den Endotrachialtubus umschließen kann. Das Band weist an seiner Innenseite Zähne auf, die sich in einem Befestigungsschlitz verhaken können. Durch das Band kann ein Endotrachialtubus an der Halterung gehalten werden.

Die Erfindung hat die Aufgabe, eine Vorrichtung zum Fixieren eines Endotrachialtubus dahin gehend zu verbessern, dass beide Teile einfach an der Vorrichtung anbringbar und von ihr lösbar sind und die Vorrichtung bei Endotrachialtuben unterschiedlicher Größen einsetzbar ist.

Die Erfindung löst die gestellte Aufgabe mittels einer Vorrichtung zum Fixieren eines Endotrachialtubus am Kopf eines Patienten mit den Merkmalen des Anspruchs 1. Somit ist eine Fixierung des Guedeltubus sichergestellt. Auch bei durch den Transport bedingten Erschütterungen oder bei Kopfbewegungen des Patienten kann der Guedeltubus fixiert werden und somit die Zufuhr von Atemluft für den Patienten sicherstellen. Außerdem kann jetzt mit einer einzigen über den Mund des Patienten legbaren Platte oder Rahmen sowohl ein Guedeltubus als auch ein Endotrachialtubus am Patienten befestigt werden. Selbstverständlich kann mit der Vorrichtung auch nur entweder ein Endotrachialtubus oder ein Guedeltubus fixiert werden. Wird nur ein Tubus benötigt, so kann die für den anderen Tubus vorgesehene öffnung auch für andere Zwecke z. B. zur Speichelabsaugung benutzt werden.

Um ein Verrutschen des Endotrachialtubus zu vermeiden, ist er mittels eines Befestigungselements in der Durchgangsöffnung fixierbar.

Vorteilhafterweise kann die Öffnung für den Guedeltubus eine umlaufende Innennut zur Aufnahme des Randes des Guedeltubus aufweisen, sodass er sicher an der Platte fixiert ist. Je nach Tiefe der umlaufenden Innennut können Guedeltuben verschiedener Größe an der Platte befestigt werden.

Besonders schnell und einfach lässt sich die Position des Endotrachialtubus korrigieren, wenn das Befestigungselement einen verstellbaren Verschluss aufweist, wie beispielsweise einen Rast- oder Klettverschluss oder ein elastisches Band. Damit ist es außerdem möglich, Endotrachialtuben mit unterschiedlichen Durchmessern in der Vorrichtung zu fixieren.

Die Platte oder der Rahmen weist eine obere und eine untere Hälfte auf, wobei die untere Plattenoder Rahmenhälfte nach unten wegklappbar ist, um den Rand des Guedeltubus in die dafür vorgesehene öffnung einzulegen.
Dies kann mittels eines seitlichen Scharniers erfolgen -

Damit die beiden voneinander wegklappbaren Rahmen- oder Plattenhälften schnell lösbar miteinander verbunden werden können, kann die obere Hälfte der Platte oder des Rahmens mittels eines Rastverschlusses an der unteren Rahmen- oder Plattenhälfte arretierbar sein.

Um den Speichel absaugen zu können, kann die Platte eine weitere Öffnung zur Sondierung des Mundraums beispielsweise mit einem Absaugrohr aufweisen. Selbstverständlich ist jedoch auch ein Absaugen durch den Guedeltubus oder durch die für seine Aufnahme vorgesehene Öffnung hindurch möglich.

Damit die Platte oder der Rahmen schnell und einfach am Patienten befestigt werden kann, kann sie mittels eines um den Kopf oder Hals führbaren Bandes mit einem Haken- oder Klettverschluss am Patienten fixiert werden. Dazu ist beispielsweise ein Klettband, ein Lochband oder ein Band mit einer Schlinge geeignet.

Nachfolgend wird ein Ausführungsbeispiel einer erfindungsgemäßen Vorrichtung zum Fixieren eines Endotrachialtubus anhand der beiliegenden Zeichnung näher erläutert.

Im Einzelnen zeigen:
- Fig. 1: eine perspektivische Ansicht einer erfindungsgemäßen Vorrichtung im geschlossenen Zustand;
- Fig. 2: eine perspektivische Ansicht der Vorrichtung im geöffneten Zustand;
- Fig. 3: eine perspektivische Ansicht eines Guedeltubus.

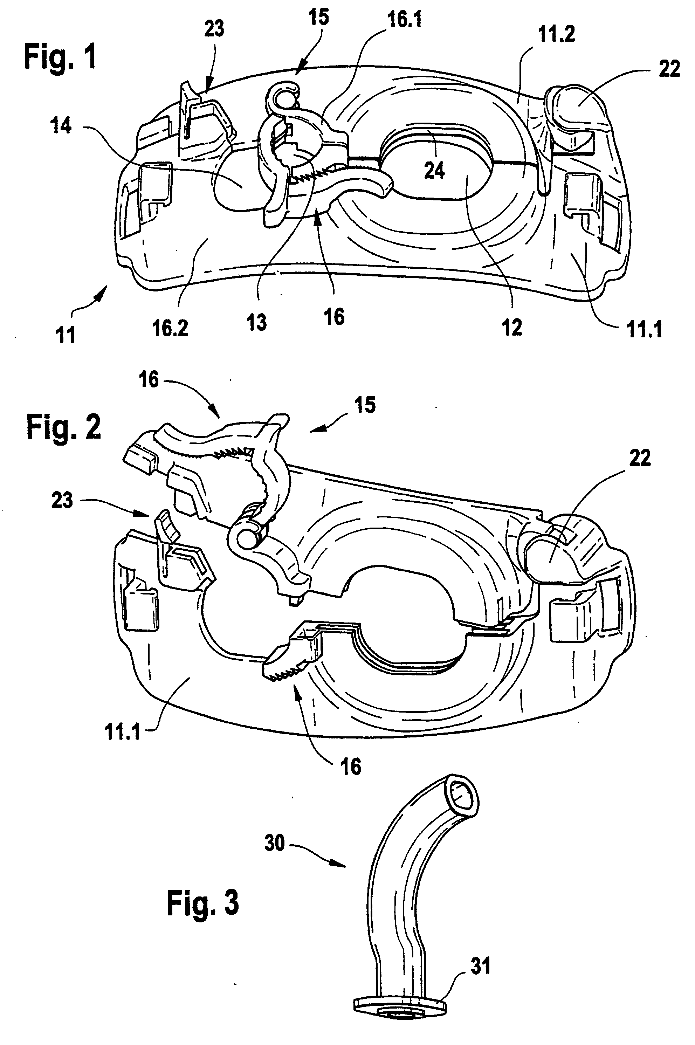

Fig. 1 zeigt eine Vorrichtung 10 zum Fixieren eines hier nicht näher dargestellten Endotrachialtubus am Kopf eines Patienten. Die Vorrichtung 10 ist hierbei in Form einer über den Mund des Patienten legbaren Platte 11 ausgebildet. Die Platte 11 weist eine öffnung 13 für den Endotrachialtubus, außerdem eine Durchgangsöffnung 12 für einen Guedeltubus 30 (Fig. 3) und eine öffnung 14 zur Sondierung des Mundraums durch verschiedene Sonden oder Katheter auf. Die Öffnung 12 für den Guedeltubus ist mit einer umlaufenden Nut 24 versehen, in die der Rand 31 des Guedeltubus 30 (Fig. 3) eingesetzt werden kann. Durch die Aufnahme des Randes 31 in die umlaufende Nut 24 ist der Guedeltubus 30 sicher im Mund fixiert und kann somit die Zunge des Patienten zuverlässig herunterdrücken. Je nach Tiefe der umlaufenden Nut 24 können in ein und dieselbe Platte 11 Guedeltuben verschiedener Größen eingespannt werden.

Der hier nicht näher dargestellte Endotrachialtubus, der durch die öffnung 13 in den Mund des Patienten geführt wird, wird durch ein Befestigungselement 15 an der Platte 11 fixiert. Das Befestigungselement 15 weist einen verstellbaren Rastverschluss 16 auf. Um ein unerwünschtes öffnen des Rastverschlusses 16 zu vermeiden, ist dieser so ausgelegt, dass er nur durch Zusammendrücken der beiden Rastverschlusselemente 16.1 und 16.2 geöffnet werden kann. Wegen der mehreren Rastnasen des Rastverschlusses 16 kann dieser Endotrachialtuben mit unterschiedlichen Durchmessern fixieren.

Die Platte 11 unterteilt sich in eine untere Hälfte 11.1 und eine obere Hälfte 11.2. Diese beiden Hälften 11.1 und 11.2 sind über ein Scharnier 22 miteinander verbunden. öffnet man den Rastverschluss 16 des Befestigungselements 15 und einen Rastverschluss 23 zwischen den Plattenhälften 11.1 und 11.2, so kann die untere Plattenhälfte 11.1 mittels des Scharniers 22 zum Einlegen des Guedeltubus 30 in die umlaufende Nut 24 nach unten weggeklappt werden (Fig. 2).

## Patentansprüche

1. Vorrichtung (10) zum Fixieren eines Endotrachialtubus am Kopf eines Patienten mit einer über den Mund des Patienten legbaren Platte (11) oder Rahmen, die eine Durchgangsöffnung (13) für den Endotrachialtubus und eine weitere Durchgangsöffnung (12) zur Fixierung eines Guedeltubus (30) aufweist, wobei der Endotrachialtubus mittels eines Befestigungselements (15) in der Durchgangsöffnung (13) fixierbar ist, **dadurch gekennzeichnet, dass** die Platte (11) oder der Rahmen eine obere Hälfte (11.2) und eine untere Hälfte (11.1) aufweist, wobei die untere Rahmen- oder Plattenhälfte (11.1) nach unten wegklappbar und das Befestigungselement (15) auf den Durchmesser des Endotrachialtubus anpassbar ist.

2. Vorrichtung (10) nach Anspruch 1, **dadurch gekennzeichnet, dass** die Öffnung (12) für den Guedeltubus (30) eine umlaufende Innennut (24) zur Aufnahme des Randes (31) des Guedeltubus (30) aufweist.

3. Vorrichtung (10) nach einem der Ansprüche 1 oder 2, **dadurch gekennzeichnet, dass** das Befestigungselement (15) einen verstellbaren Rastverschluss (16), einen Klettverschluss oder ein elastisches Band aufweist.

4. Vorrichtung (10) nach Anspruch 3, **dadurch gekennzeichnet, dass** der verstellbare Rastverschluss (16) zwei Rastverschlusselemente (16.1, 16.2) aufweist, wobei der Rastverschluss (16) durch Zusammendrücken der Rastverschlusselemente (16.1, 16.2) öffenbar ist.

5. Vorrichtung (10) nach einem der Ansprüche 1 bis 4, **dadurch gekennzeichnet, dass** die untere Rahmen- oder Plattenhälfte (11.1) mittels eines seitlichen Scharniers (22) nach unten wegklappbar ist.

6. Vorrichtung (10) nach einem der Ansprüche 1 bis 5, **dadurch gekennzeichnet, dass** die obere Hälfte (11.2) der Platte (11) oder des Rahmens mittels eines Rastverschlusses (23) an der unteren Rahmen- oder Plattenhälfte (11.1) arretierbar ist.

7. Vorrichtung (10) nach einem der Ansprüche 1 bis 6, **dadurch gekennzeichnet, dass** die Platte (11) eine weitere Öffnung (14) zum Durchstecken eines Absaugrohres aufweist.

8. Vorrichtung (10) nach einem der Ansprüche 1 bis 7, **dadurch gekennzeichnet, dass** die Platte (11) oder der Rahmen mittels eines um den Kopf oder Hals führbaren Bandes am Patienten befestigbar ist.

## Claims

1. Device (10) for securing an endotracheal tube to the head of a patient, comprising a plate (11) or frame which can be placed over the patient's mouth and which has a through aperture (13) for the endotracheal tube and a further through aperture (12) for securing a Guedel's tube (30), the endotracheal tube being fixable in the through aperture (13) by means of a fixing element (15), **characterised in that** the plate (11 ) or frame includes an upper half (11.2) and a lower half (11.1), the lower half (11.1) of the frame or plate being able to be folded away downwardly and the fixing element (15) being adaptable to the diameter of the endotracheal tube.

2. Device (10) according to claim 1, **characterised in that** the aperture (12) for the Guedel's tube (30) includes an internal peripheral groove (24) for receiving the edge (31) of the Guedel's tube (30).

3. Device (10) according to either of claims 1 and 2, **characterised in that** the fixing element (15) comprises an adjustable latching closure (16), a Velcro-type closure or an elastic strap.

4. Device (10) according to claim 3, **characterised in that** the adjustable latching closure (16) includes two latching closure elements (16.1, 16.2), the latching closure (16) being openable by pressing together the latching closure elements (16.1, 16.2).

5. Device (10) according to any one of claims 1 to 4, **characterised in that** the lower half (11.1) of the frame or plate can be folded away downwardly by means of a lateral hinge (22).

6. Device (10) according to any one of claims 1 to 5, **characterised in that** the upper half (11.2) of the plate (11) or frame is lockable to the lower half (11.1) of the frame or plate by means of a latching closure (23).

7. Device (10) according to any one of claims 1 to 6, **characterised in that** the plate (11) includes a further aperture (14) through which a suction tube can be pushed.

8. Device (10) according to any one of claims 1 to 7, **characterised in that** the plate (11) or frame can be secured to the patient by means of a strap which can be passed around the head or neck.

## Revendications

1. Dispositif (10) pour la fixation d'un tube endotrachéal à la tête d'un patient, avec une plaque (11) ou un châssis applicable sur la bouche du patient, qui comporte un orifice traversant (13) pour le tube endotrachéal et un autre orifice traversant (12) pour la fixation d'un tube de Guedel (30), le tube endotrachéal pouvant être fixé dans l'orifice traversant (13) au moyen d'un élément de fixation (15), **caractérisé en ce que** la plaque (11) ou le châssis comporte une moitié supérieure (11.2) et une moitié inférieure (11.1), la moitié inférieure de châssis ou de plaque (11.1) étant mobile vers le bas et l'élément de fixation (15) étant adaptable au diamètre du tube endotrachéal.

2. Dispositif (10) selon la revendication 1, **caractérisé en ce que** l'orifice (12) pour le tube de Guedel (30) comporte une rainure intérieure périphérique (24) pour la réception du bord (31) du tube de Guedel (30).

3. Dispositif (10) selon une des revendications 1 ou 2, **caractérisé en ce que** l'élément de fixation (15) comporte une fermeture par crantage réglable (16), une fermeture autoagrippante ou une bande élastique.

4. Dispositif (10) selon la revendication 3, **caractérisé en ce que** la fermeture par crantage réglable (16) comporte deux éléments de fermeture par crantage (16.1, 16.2), la fermeture par crantage (16) pouvant être ouverte par pression ensemble des éléments de fermeture par crantage (16.1, 16.2).

5. Dispositif (10) selon une des revendications 1 à 4, **caractérisé en ce que** la moitié inférieure de châssis ou de plaque (11.1) est mobile vers le bas au moyen d'une charnière latérale (22).

6. Dispositif (10) selon une des revendications 1 à 5, **caractérisé en ce que** la moitié supérieure (11.2) de la plaque (11) ou du châssis peut être bloquée contre la moitié inférieure de châssis ou de plaque (11.1) au moyen d'une fermeture par crantage (23).

7. Dispositif (10) selon une des revendications 1 à 6, **caractérisé en ce que** la plaque (11) comporte un autre orifice (14) pour le passage d'un tuyau d'aspiration.

8. Dispositif (10) selon une des revendications 1 à 7, **caractérisé en ce que** la plaque (11) ou le châssis peut être fixé au patient au moyen d'une bande passant autour de la tête ou du cou.
